# EUROPEAN PATENT APPLICATION

(11) **EP 2 899 273 A1**
(43) Date of publication of application: **29.07.2015**
(21) Application number: 13838813.7
(22) Date of filing: 19.09.2013
(51) Int. Cl.: C12N 15/09, C12N 1/19, C12N 1/21, C12N 9/02, C12N 9/88, C12P 7/18

(54) **GENE, MICROORGANISM, CONVERSION METHOD, AND PRODUCTION METHOD**

(30) Priority: 24.09.2012 JP 2012209982
(71) Applicant: Showa Denko K.K., Tokyo 105-8518 (JP)
(72) Inventor: AOKI, Hirobumi, Tokyo 105-8518 (JP); KOKIDO, Yuzuru, Tokyo 105-8518 (JP); YONEDA, Tadashi, Tokyo 105-8518 (JP)
(74) Representative: Strehl Schübel-Hopf & Partner
(86) International application number: PCT/JP2013/075296
(87) International publication number: WO 2014/046178

(57) **Abstract**

A gene described in any of undermentioned (a) - (c), wherein the gene in combination with a gene that codes enoyl-CoA hydratase is able to provide a microbe or a culture of the microbe with an ability to convert 3-hydroxybutyryl-CoA into 4-hydroxybutyryl-CoA or 4-hydroxybutyryl-CoA into 3-hydroxybutyryl-CoA.
(a) a gene that has a base sequence of sequence number 1
(b) a gene that has a base sequence such that one or more bases are deleted, substituted, or added in a base sequence of sequence number 1, wherein the gene has a base sequence with an identity greater than or equal to 90% with respect to the base sequence of sequence number 1
(c) a gene that hybridizes with a gene that has a base sequence complimentary with a gene that has a base sequence described in sequence number 1 on a stringent condition

## Description

### TECHNICAL FIELD

The present invention relates to a gene, a microbe, a conversion method, and a manufacturing method.

### BACKGROUND ART

In recent years, attention is paid to a compound manufacturing process with a renewable source as a raw material from the viewpoint of depletion of fossil resource, a countermeasure for global warming, or the like. In particular, a so-called bio-refinery has widely been studied wherein a variety of compounds as raw materials for a polymer or compounds as raw materials for a chemical product are manufactured in biochemical processes with biomass as raw materials.

For compounds that are expected as raw material conversion of biomass, there are provided butanediols (that refer to 1,3-butanediol and 1,4-butanediol). For example, 1,4-butanediol is used as a synthetic raw material for a precision organic chemical product, a monomer unit of a polyester and an engineering plastic, or the like, and 1,3-butanediol is used as an organic solvent, a substrate for cosmetic product, or the like. In a case of any of 1,4-butanediol and 1,3-butanediol, a need for a biochemical process with a renewable source such as biomass as a raw material is increased.

For manufacturing methods for a butanediol that use a biochemical process, there are provided, for example, methods described in patent documents 1 to 3 and non-patent document 1.

### PRIOR ART DOCUMENTS

[Patent Document 1] Japanese Patent No. 4380704 specification
[Patent Document 2] International Publication No. 2008/115840 official gazette
[Patent Document 3] International Publication No. 2010/127319 official gazette
[Non-patent document 1] Harry Yim et al., Metabolic engineering of Escherichia coli for direct production of 1,4-butanediol, Nature Chemical Biology, 7, 445-452 (2011).

### DISCLOSURE OF THE INVENTION

### PROBLEMS TO BE SOLVED BY THE INVENTION

However, methods described in patent documents 1 to 3 and non-patent document 1 are complicated processes.

Against a problem as described above, there is provided a new manufacturing method for 1,4-butanediol that is able to obtain 1,4-butanediol economically. Furthermore, there are provided a conversion method associated with such a manufacturing method, and a gene and a microbe that are applicable to such a manufacturing method and such a conversion method.

### MEANS FOR SOLVING THE PROBLEM

The present inventors actively studied to solve a problem as described above, and as a result, found that a particular gene was effective, so that the present invention was achieved. That is, the present invention included the following.
[1] A gene described in any of undermentioned (a) - (c), wherein the gene in combination with a gene that codes enoyl-CoA hydratase is able to provide a microbe or a culture of the microbe with an ability to convert 3-hydroxybutyryl-CoA into 4-hydroxybutyryl-CoA or 4-hydroxybutyryl-CoA into 3-hydroxybutyryl-CoA.
   (a) a gene that has a base sequence of sequence number 1
   (b) a gene that has a base sequence such that one or more bases are deleted, substituted, or added in a base sequence of sequence number 1, wherein the gene has a base sequence with an identity greater than or equal to 90% with respect to the base sequence of sequence number 1
   (c) a gene that hybridizes with a gene that has a base sequence complimentary with a gene that has a base sequence described in sequence number 1 on a stringent condition
[2] The gene as described in [1], wherein the microbe is Escherichia coli, a yeast, a corynebacterium, or a clostridium.
[3] A microbe that includes the gene as described in [1] and a gene that codes enoyl-CoA hydratase.
[4] The microbe as described in [3], further including a gene that codes acyl-CoA reductase.
[5] A conversion method, wherein the microbe as described in [3] or a culture of the microbe is used to convert 3-hydroxybutyryl-CoA into 4-hydroxybutyryl-CoA or 4-hydroxybutyryl-CoA into 3-hydroxybutyryl-CoA.
[6] A manufacturing method, wherein the microbe as described in [4] or a culture of the microbe is used to manufacture 1,4-butanediol.
[7] The manufacturing method as described in [6], wherein the microbe includes a gene that codes at least one of acetoacetyl-CoA synthase or acetoacetyl-CoA reductase.
[8] The manufacturing method as described in [6], wherein 1,4-butanediol is manufactured from acetyl-CoA via 3-hydroxybutyryl-CoA.

### EFFECTS OF THE INVENTION

It is possible to provide an economical manufacturing method for 1,4-butanediol. Furthermore, it is possible to provide a conversion method associated with such a manufacturing method, and a gene and a microbe that are applicable to such a manufacturing method and such a conversion method.

### BRIEF DESCRIPTION OF THE DRAWINGS

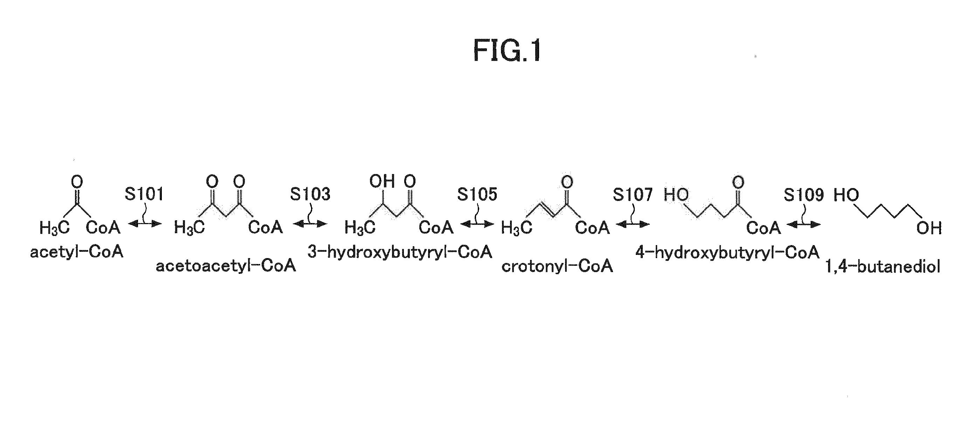

[FIG. 1] is a schematic diagram for illustrating a conversion method between 3-hydroxybutyryl and 4-hydroxybutyryl according to the present embodiment.

### BEST MODE FOR IMPLEMENTING THE INVENTION

The present invention will be described below, by using FIG. 1. Here, "CoA" means coenzyme A in the present specification.

### (A gene)

First, a gene in the present embodiment will be described that is able to provide, in combination with a gene that codes enoyl-CoA hydratase, a microbe or a culture of such a microbe with an ability to convert 3-hydroxybutyryl-CoA into 4-hydroxybutyryl-CoA or 4-hydroxybutyryl-CoA into 3-hydroxybutyryl-CoA.

A gene in the present embodiment is a gene that has a base sequence of sequence number 1 and a homolog thereof. A gene in the present embodiment in combination with a gene that codes an enzyme enoyl-CoA hydratase is expressed in a microbial body by means of transformation or the like, and thereby, is able to be applied to, for example, a conversion method between 3-hydroxybutyryl and 4-hydroxybutyryl or a manufacturing method for 1,4-butanediol.

A homolog in the present embodiment includes an ortholog and a paralog. An ortholog refers to a set of corresponding genes among species generated from a gene of a common ancestor by means of speciation and enzymes obtained from such genes. A paralog refers to a set of corresponding genes among species generated by means of not speciation but gene duplication in an identical species and enzymes obtained from such genes. A homolog refers to genes that have an identity in sequences thereof, regardless of an ortholog or a paralog, and enzymes obtained from such genes.

More specifically, a homolog gene for a gene that has a base sequence described in sequence number 1 includes a gene that has a base sequence with an identity greater than or equal to 90%, preferably an identity greater than or equal to 95%, with respect to a base sequence described in sequence number 1, and more preferably, a gene wherein one or several bases of such a gene are deleted, substituted, or added.

Furthermore, a homolog gene described previously includes a gene that hybridizes with a gene that has a base sequence complementary with a gene that has a base sequence described in sequence number 1, on a stringent condition. Specifically, it is possible to acquire a gene (or an enzyme obtained by transformation caused by such a gene) by applying a homology retrieval program (for example, BLAST or FASTA) to a publicly-known data base, or based on an ordinary method such as hybridization or polymerase chain reaction (PCR) on a stringent condition that uses a probe that is composed of at least a portion of an identified gene (DNA that is composed of a base sequence complementary with DNA that is composed of a base sequence of such a gene). Furthermore, it is possible for a person(s) skilled in the art to execute self-design by substituting a base sequence or the like. Here, for a stringent condition referred herein, there is provided, for example, a condition for executing hybridization as described in a non-patent document of Molecular Cloning - A LABORATORY MANUAL THIRD EDITION (Joseph Sambrook, David W. Russell., Cold Spring Harbor Laboratory Press). Specifically, a hybridizing condition is to be retained with a probe in a solution that includes 6 x SSC (a composition of 1 x SSC: 0.15 M of sodium chloride, 0.015 M of sodium citrate, pH: 7.0), 0.5% of SDS, 5 x Denhardt's solution, and 100 mg/mL of herring sperm DNA, at a constant temperature of 65 °C for 8 - 16 hours.

### (A microbe)

An example of a transformation host microbe is not particularly limited that is able to introduce a gene in the present embodiment and a gene that codes an enzyme enoyl-CoA hydratase therein, as long as it is possible to apply a gene recombination technique to such a microbe. From the viewpoint of industrial availability, there is provided Escherichia coli, a yeast, a coryneform bacteria, or a clostridial bacteria, as a specific example. For a yeast, there is provided Saccharomyces cerevisiae, Schizosaccharomyces pombe, Kluyveromyces lactis, Kluyveromyces marxianus, or the like. For a coryneform bacteria, there is provided, Corynebacterium glutamicum, Corynebacterium efficiens, Brevibacterium divaricatum, Brevibacterium saccharolyticum, Brevibacterium immariophilum, Brevibacterium lactofermentum, Brevibacterium roseum, Brevibacterium flavum, Brevibacterium thiogenitalis, Corynebacterium acetoacidophilum, Corynebacterium acetoglutamicum, Corynebacterium callunae, Corynebacterium lilium, Corynebacterium mellassecola, Microbacterium ammoniaphilum, or the like. For a clostridial bacteria, there is provided Clostridium kluyveri, Clostridium acetobutylicum, Clostridium aminobutyricum, Clostridium beijerinckii, Clostridium saccharoperbutylacetonicum, or the like. Among these, it is preferable to use Escherichia coli, Saccharomyces cerevisiae, Schizosaccharomyces pombe, or Corynebacterium glutamicum, because transformation thereof is easy, and it is more preferable to use Escherichia coli.

Furthermore, a transformation microbe in the present embodiment may be used as a cultured bacterial body of a microbe itself or in each kind of form of a culture thereof. Specifically, a culture of a microbe in the present embodiment includes a suspension of a cultured bacterial body of a microbe in a medium such as a culture medium or a buffer solution, a cell-free extract from a cultured bacterial body of a microbe, and further, a processed substance such as a component that catalyzes such a reaction and is concentrated, purified, or extracted from such a cell-free extract. A culture of a microbe in the present embodiment further includes a processed substance of a microbe as described previously that is fixed on a hardly soluble carrier. For such a fixation carrier, there is provided a compound that forms a solid content that is hardly soluble in water and encapsulates a bacterial body of a microbe or a processed substance thereof as described previously, such as a polyacrylamide, a polyvinyl alcohol, a poly-N-vinylformamide, a polyallylamine, a polyethyleneimine, a methylcellulose, a glucomannan, an alginate, carrageenan, or the like, or further, a copolymer or cross-linked substance thereof, or the like. These are such that one kind thereof may be used singly or two or more kinds thereof may be mixed and used. Furthermore, it is also possible to use, as a culture of a microbe, a microbe or an extract or extract component thereof held on a substance that is preliminarily formed as a solid, such as an activated carbon, a porous ceramic, a glass fiber, a porous polymer molded body, or a nitrocellulose film.

### (A conversion method)

As described previously, a gene in the present embodiment in combination with a gene that codes an enzyme enoyl-CoA hydratase is expressed in a microbial body by means of transformation or the like, and thereby, is able to be applied to a conversion method between 3-hydroxybutyryl and 4-hydroxybutyryl.

FIG. 1 illustrates a schematic diagram for illustrating a conversion method between 3-hydroxybutyryl and 4-hydroxybutyryl according to the present embodiment. As 3-hydroxybutyryl-CoA is supplied to a microbial body as described later, rearrangement reaction of a hydroxyl group (OH group) is caused by an action of a gene as described previously so that 3-hydroxybutyryl-CoA is converted into 4-hydroxybutyryl-CoA (S105 and S107). Furthermore, as 4-hydroxybutyryl-CoA is supplied into such a microbial body, a rearrangement reaction of an OH group is caused by an action of a gene as described previously, so that 4-hydroxybutyryl-CoA is converted into 3- hydroxybutyryl-CoA.

A result of translation of a gene indicated by sequence number 1 into an amino acid sequence is homologous with a result of translation of an abfD gene derived from Clostridium aminobutyricum that is able to be referred to by accession No. AJ250267 of the Gen Bank. A detail of an enzyme that is coded by an anfD gene is described in a non-patent document of "Fermentation of 4-aminobutyrate by Clostridium aminobutyricum: cloning of two genes involved in the formation and dehydration of 4-hydroxybutyryl-CoA, Archives of Microbiology, 174(3) 189-199 (2000)" or the like. From a translated sequence and a reaction provided by a gene as described previously, it is considered that a gene indicated by sequence number 1 codes an enzyme that has an activity of 4-hydroxybutyryl-CoA dehytratase. However, a conversion function between 3-hydroxybutyryl and 4-hydroxybutyryl does not act in a case where a recombinant that includes an abfD gene sequence is used, as described later for a comparative example. That is, in the present embodiment, it is considered that a conversion function between 3-hydroxybutyryl and 4-hydroxybutyryl is developed by coexpression of a gene that has a base sequence of sequence number 1 and another gene in a recombinant. Here, a gene indicated by sequence number 1 in the present embodiment and an abfD gene as described previously are such that coded amino acid sequences are homologous but homology in base sequences thereof in a used gene information processing software (GENETYX; produced by GENETYX CORPORATION) is 75% and greatly different. In the present embodiment wherein genes with greatly different base sequences are applied thereto, it is possible to provide a conversion method that has a high conversion efficiency between 3-hydroxybutyryl and 4-hydroxybutyryl.

### (A manufacturing method for 1,4-butanediol)

3-hydroxybutyryl-CoA and/or 4-hydroxybutyryl-CoA that is/are obtained by a conversion method as described previously is/are further combined with another reaction in such a manner that an acyl-CoA moiety thereof is a reaction site, and thereby, it is possible to provide a precursor for manufacturing a butanediol. Therefore, it is possible to apply a gene in the present embodiment to a manufacturing method for a butanediol (for example, 1,4-butanediol) with a good efficiency.

FIG. 1 also illustrates a schematic diagram for illustrating a manufacturing method for 1,4-butanediol according to the present embodiment. In the present embodiment, a gene indicated by sequence number 1, a gene that codes an enzyme enoyl-CoA hydratase, and further, a gene that codes acyl-CoA reductase are expressed in a microbial body. As 3-hydroxybutyryl-CoA is supplied into such a microbial body on a condition that these genes are expressed, conversion into 4-hydroxybutyryl-CoA is caused first (S105 and S107). Subsequently, conversion into 1,4-butanediol is caused by an action of a gene that codes an acyl-CoA reductase (S109). Furthermore, 3-hydroxybutyryl-CoA is converted into 1,3-butanediol that is not illustrated in the figure, by an action of a gene that codes an acyl-CoA reductase.

Here, in the present embodiment, an enzyme or a group of a series of enzymes as described in the following is/are co-expressed in a microbial body transformed by means of gene recombination so that a reaction is caused to proceed. A gene that codes each enzyme is inserted into an arbitrary vector individually or as a series of clusters and a host microbe is transformed thereby. An obtained transformed body is cultured in a culture medium with an appropriate carbon source, for example, a carbon source of glucose, and thereby, each gene is expressed. In a case of a gene that is capable of being configured and expressed in a host, a transformed body is cultured in a culture medium and thereby, such a gene is expressed. On the other hand, in a case where each gene is configured under a control of a regulator arranged on a vector, an induction substrate is added to transfer to an inductive environment, and thereby, each coded gene is expressed. Here, culturing in the present embodiment includes all of culturing conditions for normal microbe culturing, and further, a culturing step means culturing for a period of time and on a condition that are enough for a microbe to manufacture a butanediol.

A manufacturing method for 3-hydroxybutyryl-CoA or 4-hydroxybutyryl-CoA will be described that is a substrate in a manufacturing method for a butanediol in the present embodiment. First, an enzyme reaction (S101) that uses a gene that codes thiolase (EC number 2. 3. 1. 9) or a homolog thereof is utilized so that one molecule of CoA is eliminated from two molecules of acetyl-CoA under a supply of acetyl-CoA to afford acetoacetyl-CoA. Here, acetyl-CoA is obtained through a known route such as a glycolysis system. Then, it is possible to obtain 3-hydroxybutyryl-CoA by an enzyme reaction that uses acetoacetyl-CoA reductase (EC number 1. 1. 1. 36), 3-hydroxybutyryl-CoA dehydrogenase (EC number 1. 1. 1. 35), 3-hydroxyacyl-CoA dehydrogenase (EC number 1. 1. 1. 157), or homologs thereof that has/have an activity for reducing an obtained acetoacetyl-CoA to afford 3-hydroxybutyryl-CoA. Here, it is possible to obtain (R)-3-hydroxybutyryl-CoA in a case where acetoacetyl-CoA reductase (EC number: 1. 1. 1. 36) is used, and it is possible to obtain (S)-3-hydroxybutyryl-CoA in a case where 3-hydroxybutyryl-CoA dehydrogenase (EC number 1. 1. 1. 35) or 3-hydroxyacyl-CoA dehydrogenase (EC number 1. 1. 1. 157) is used.

Furthermore, propanoate CoA-transferase (EC number 2. 8. 3. 1) is known as an enzyme that transfers CoA to 3-hydroxybutanoic acid directly. 3-hydroxybutanoic acid is supplied to a microbe or a culture that includes such a microbe under expression of a gene that codes propanoate CoA-transferase or a homolog thereof and under supply of acetyl-CoA that is a donor for a CoA transfer reaction, and thereby, it is possible to produce 3-hydroxybutyryl-CoA. Furthermore, 4-hydroxybutyryl-CoA; CoA mutase as described in a non-patent document of "Molecular analysis of the anaerobic succinate degradation pathway in Clostridium kluyveri, Journal of Bacteriology, 178 (3), 871 - 880 (1996)" or the like is known as an enzyme that transfers CoA to 4-hydroxybotanic acid. 4-hydroxybutanoic acid is supplied to a microbe or a culture of such a microbe under expression of a gene that codes such an enzyme or a homolog thereof and under supply of acetyl-CoA that is a donor for a CoA transfer reaction, and thereby, it is possible to obtain 4-hydroxybutyryl-CoA.

### (Anther enzyme and a gene that codes such an enzyme)

Next, a specific example of each enzyme described above and a gene that codes such an enzyme will be described.

### [A gene that codes enoyl-CoA hydratase]

Enoyl-CoA hydratase that is used in the present embodiment is not particularly limited as long as an enzyme catalyzes a reaction that dehydrates 3-hydroxybutyryl-CoA to produce crotonyl-CoA.

For a specific example of a gene that codes an enzyme that catalyzes a reaction that produces crotonyl-CoA from (S)-3-hydroxybutyryl-CoA, there is provided a gene that codes enoyl-CoA hydratase (EC number: 4. 2. 1. 17) or a homolog thereof. For a detail of a gene that codes enoyl-CoA hydratase (EC number: 4. 2. 1. 17), it is possible to refer to a non-patent document of "The complete stereochemistry of the enzymatic dehydration of 4-hydroxybutyryl coenzyme A to crotonyl coenzyme. A., Friedrich P, Darley DJ, Golding BT, Buckel W., Angewandte Chemie International Edition, 2008 47 (17) 3254 - 3257 (2008)" or the like. Furthermore, for a gene that codes an enzyme that catalyzes a reaction that produces crotonyl-CoA from (R)-3-hydroxybutyryl-CoA, there is provided a gene that codes enoyl-CoA hydratase (EC number: 4. 2. 1. 119), a homolog thereof, or the like. For a detail of a gene that codes enoyl-CoA hydratase (EC number: 4. 2. 1. 119), it is possible to refer to a non-patent document of "Metabolism of poly-beta-hydroxybutyrate. II. Enzymatic synthesis of D-(-)-beta hydroxybutyryl coenzyme A by an enoyl hydrase from Rhodospirillum rubrum., Moskowitz GJ, Merrick JM. Journal Biochemistry., 8 2748 - 2755 (1969)" or the like.

As described previously, it is possible for acetoacetyl-CoA reductase that catalyzes a reaction that produces 3-hydroxybutyryl-CoA from acetoacetyl-CoA to produce an (R)-body and/or (S)-body of 3-hydroxybutyryl-CoA depending on a kind of an enzyme to be applied. Furthermore, enoyl-CoA hydratase that catalyzes a reaction that produces crotonyl-CoA from 3-hydroxybutyryl-CoA also has a similar selectivity of an optical isomer. That is, it is possible for a manufacturing method for a butanediol according to the present embodiment to combine optical selectivities of acetoacetyl-CoA reductase and enoyl-CoA hydratase suitably, and hence, it is advantageous from the viewpoint of a yield of a reaction that produces crotonyl-CoA from acetoacetyl-CoA or the like.

### [A gene that codes acyl-CoA reductase]

It is possible to arbitrarily select acyl-CoA reductase gene that is used in the present invention as long as an enzyme is coded that catalyzes a reaction that reduces a CoA site of 4-hydroxybutyryl-CoA to produce 1,4-butanediol. Specifically, it is possible to use a gene that codes an oxidoreductase that is included in a group of EC number: 1. 2. 1 and acts on an aldehyde body (for example aldehyde dehydrogenase (EC number 1. 2. 1. 10)) or a homolog thereof. For a detail of an enzyme as described previously, it is possible to refer to a non-patent document of "Purification, properties, and kinetic mechanism of coenzyme A-linked aldehyde dehydrogenase from Clostridium kluyveri., Journal Arch. Biochem. Biophys. 203 663 - 75 (1980)" or the like.

It is possible for an aldehyde body to obtain 1,4-butanediol because an alcohol is induced by alcohol dehydrogenase in a host. For a variation example of the present embodiment, it is possible to obtain 1,4-butanediol even in a case where alcohol dehydrogenase that has a 4-hydroxybutanal reduction activity is combined and co-expressed. Furthermore, as another variation example of the present embodiment, it is also possible to obtain 1,4-butanediol from 4-hydroxybutyryl-CoA by using a gene that codes an aldehyde/alcohol reductase that has a hybrid function or a homolog thereof. For a detail of an enzyme that has such a hybrid function, it is possible to refer to a non-patent document of "Molecular Characterization and Transcriptional Analysis of adhE2, the Gene Encoding the NADH-Dependent Aldehyde/Alcohol Dehydrogenase Responsible for Butanol Production in Alcohologenic Cultures of Clostridium acetobutylicum ATCC 824., Journal of Bacteriology, 184 (3) 821-830 (2002)" or the like.

### (A culturing condition)

For example, a reaction in the present invention is most conveniently achieved in such a manner that a transformed body is cultured in a nutritive medium such as an LB medium at a temperature of 15 °C - 40 °C, desirably 18 °C - 37 °C for about 24 hours, subsequently implanted to a medium with a normal carbon source, for example, a carbon source that is 0.01 - 50%, desirably 0.1 - 30%, of glucose, and continuously cultured at a similar temperature for about 1 hour - 200 hours, wherein a butanediol is stored in a culture solution in such a process. Furthermore, a carbon source may be added continuously or intermittently depending on consumption of a carbon source that is caused by proliferation of a bacteria or proceeding of a reaction, and in such a case, a concentration of a carbon source in a reaction fluid is not limited to one described previously.

As a medium carbon source for culturing a microbe, it is possible to use, for example, a saccharide such as glucose, sucrose, or fructose, a polyol such as glycerol, an organic substance such as ethanol, acetic acid, citric acid, succinic acid, lactic acid, benzoic acid, or a fatty acid, or an alkali metal salt thereof, an aliphatic hydrocarbon such as an n-paraffin, an aromatic hydrocarbon, or a natural organic substance such as peptone, meat extract, fish extract, soybean flour, or bran, singly or in combination thereof, at a concentration of normally 0.01%-30%, desirably about 0.1% - 20%.

For a medium nitrogen source for culturing a microbe, it is possible to use, for example, an inorganic nitrogen compound such as ammonium sulfate, ammonium phosphate, sodium nitrate, or potassium nitrate, a nitrogen-containing organic substance such as urea or uric acid, or a natural organic substance such as peptone, meat extract, fish extract, or soybean flour, singly or in combination thereof, at a concentration of normally 0.01%-20%, desirably about 0.1% - 10%.

Moreover, it is possible to add a phosphate such as potassium dihydrogen phosphate, or a metal salt such as magnesium sulfate, ferrous sulfate, calcium acetate, manganese chloride, copper sulfate, zinc sulfate, cobalt sulfate, or nickel sulfate, as necessary, for growth of a bacteria or improvement of an enzyme activity. A concentration for addition thereof is different depending on a culturing condition, and normally, is 0.01% - 5% for a phosphate, 10 ppm - 1% for a magnesium salt, or about 0.1 ppm - 1,000 ppm for other compounds. Furthermore, for a source of supply for a vitamin, an amino acid, a nucleic acid, or the like, it is possible to add, for example, about 1 ppm - 100 ppm of yeast extract, casamino acid, or a yeast nucleic acid, depending on a selected medium, for growth of a bacteria or improvement of an enzyme activity.

It is desirable to adjust a pH of a medium to 4.5 - 9, desirably 5 - 8. Furthermore, it is useful to fractionate from a culture solution by a method such as centrifugation or membrane filtration, and again suspend and react in water that includes a reaction raw material, physiological saline, a buffer that has a pH adjusted to be comparable to a pH for culturing and composed of phosphoric acid, acetic acid, boric acid, tris(hydroxymethyl)aminomethane, or the like, or a salt thereof, or the like, a microbial or bacterial body that is preliminarily cultured in a medium as described previously, in order to reduce an impurity in a reaction fluid and simplify subsequent fractionation of a product. Although a pH during a reaction is able to be normally retained in a case where a buffer with a sufficient concentration is used, it is desirable to be adjusted appropriately by using sodium hydroxide, ammonia, or the like so as to provide a similar pH in a case where deviation from a pH as described above is caused by proceeding of a reaction.

It is possible to execute separation, recovery, and purification of a butanediol produced in a reaction fluid by using a general means of separation, recovery, and purification of an organic compound, after a bacterial body is eliminated from such a reaction fluid by means of centrifugation at a point of time when an amount of a produced butanediol reaches a substantial amount, or for such a reaction fluid directly. For example, extraction from a filtrate wherein a bacterial body and the others are eliminated from a culture solution is executed by using an appropriate organic solvent. Such an extract is directly distilled away, is further extracted again with an appropriate solvent, purified by using silica gel chromatography or the like, or is subjected to multistage distillation or the like, and thereby, a butanediol is obtained at a high purity.

In a case where a butanediol is stored during a reaction and thereby a reaction rate is lowered, a method is preferable that adds water, physiological saline, a reaction buffer, or the like, into a reaction fluid depending on a concentration of a product to execute continuous dilution thereof. Furthermore, at a point of time when a reaction rate is lowered, a bacteria is fractionated and a supernatant is recovered as a product solution, wherein a fractionated bacteria is again returned to a solution or suspension that includes a reaction raw material and thereby it is possible to recover a reaction rate. It is possible to execute such an operation continuously or even batch-wise by using a centrifuge, a separation film, or the like.

### PRACTICAL EXAMPLES

The present invention will be described in more detail below by using practical examples, wherein the present invention is not limited to under-mentioned practical examples.

### (Practical Example 1)

A blunt end fragment of a base sequence indicated by sequence number 3 (that corresponded to 4-hydroxybutyryl-CoA; CoA mutase) was totally synthesized to add GGATCC (that corresponded to a BamHI site) to a 5'-end and CTCGAG (that corresponded to an XhoI site) to a 3'-end by an ordinary method and inserted into an SmaI site in multi-cloning sites of pUC18 to obtain a plasmid. An obtained plasmid was treated with a restriction enzyme to obtain a BamHI - XhoI fragment that contained a region of sequence 3. An obtained gene fragment was inserted into a BamHI - XhoI site in multi-cloning sites of an expression vector pET17b (produced by Novagen, Inc.) by an ordinary method to obtain a plasmid pETSD3.

A blunt end fragment of a base sequence indicated by sequence number 1 was totally synthesize to add CATATG (that corresponded to an NdeI site) to a 5'-end and AAGCTT (that corresponded to a HindIII site) to a 3'-end by an ordinary method and inserted into an SmaI site in multi-cloning sites of pUC18 to obtain a plasmid. An obtained plasmid was treated with a restriction enzyme to obtain an NdeI - HindIII fragment that contained a region of sequence 1. An obtained gene fragment was inserted into an NdeI-HindIII site in multi-cloning sites of an expression vector pET17b (produced by Novagen, Inc.) by an ordinary method to obtain a plasmid pETSD1. An obtained pETSD1 as a template was annealed upstream of a pE17b-vector-derived T7 promoter of pETSD1, PCR was executed by using a primer with GGATCC (that corresponded to BamHI site) further added at 5'-side and a primer complementing a 3'-end of sequence 1 of pETSD1 and provided with GAGCTC (that corresponded to an SacI site) further added at a 5'-side, and an obtained amplified product was treated with SacI to obtain fragment 1.

A blunt end fragment of a base sequence indicated by sequence number 2 (that corresponded to enoyl-CoA hydratase) was totally synthesized to add CATATG (that corresponded to an NdeI site) to a 5'-end and CTCGAG (that corresponded to an XhoI site) to a 3'-end by an ordinary method and inserted into an SmaI site in multi-cloning sites of pUC18 to obtain a plasmid. An obtained plasmid was treated with a restriction enzyme to obtain an NdeI-XhoI fragment that contained a region of sequence 2. An obtained gene fragment was inserted into an NdeI - XhoI site in multi-cloning sites 2 of an expression vector pETDuet (produced by Novagen, Inc.) by an ordinary method to obtain a plasmid pEDSD02. An obtained plasmid pEDSD02 as a template was annealed upstream of a pETDuet-vector-derived T7Lac promoter of pEDSD02, PCR was executed by using a primer with GAGCTC (that corresponded to an SacI site) further added at 5'-side and a primer complementing a 3'-end of sequence 1 of pEDSD01 and provided with a complementary sequence of GGATCC (that corresponded to a BamHI site) further added at a 5'-side, and an obtained amplified product was treated with SacI to obtain fragment 2.

Ligation of fragment 1 and fragment 2 was executed at both SacI sites thereof by an ordinary method to obtain fragment 3 that contained fragment 1 and fragment 2. Then, PCR was executed by using fragment 3 and a primer that was preliminarily designed in such a manner that 15 bp of upstream and downstream sides of a BamHI site of pETSD3 (that includes a BamHI site of fragment 3) were added to outsides or both ends of fragment 3, respectively. An obtained amplified product and a BamHI-treated fragment of pETSD3 were coupled in In-Fusion HD Cloning Kit (produced by TAKARA BIO INC.) to obtain a plasmid pETSD123 that contained fragment 3, fragment 1, and fragment 2.

An Escherichia coli Rosetta Blue (DE3) strain (produced by Novagen, Inc.) was transformed by using an obtained plasmid pETSD123 and an ordinary method.

A transformed body pETSD123/Rosetta Blue (DE3) was aerobically cultured in 5 mL of an LB medium that contained 100 mg/L of ampicillin and 32 mg/L of chloramphenicol at 37 °C for 12 hours. 0.1 mL of a culture solution was implanted in 5 mL of evaluation medium 1 that contained 100 mg/L of ampicillin, 32 mg/L of chloramphenicol, 0.2 mM of IPTG, and further 0.3% of sodium 4-hydroxybutanoate, and aerobically cultured at 30 °C for 48 hours.

A composition of evaluation medium 1 was:
2% glucose;
1% peptone (produced by Difco Inc.);
0.5% yeast extract (produced by Difco Inc.);
2.4% K₂HPO₄;
0.6% KH₂PO₄;
600 ppm iron citrate; and
100 ppm riboflavin / distilled water (pH 7.2).

Here, evaluation medium 1 was provided in such a manner that all of compositions described above were admixed, stirred at ordinary temperature for 2 hours, and subsequently filtration-sterilized by using a filter with φ 0.45 µm.

Sodium 4-hydroxybutanoate used in the present embodiment was synthesized by an under-mentioned method based on a synthesis method described in International Publication No. 2010/068953 official gazette. While 50 mL of methanol with 0.1 M of NaOH dissolved therein was stirred, 0.1 M of γ-butyrolactone was dropped therein. After stirring for 1 hour, a solution was dried at 60 °C for 3 hours to obtain a colorless crystal. Pure water was added into an obtained crystal to provide an aqueous solution and an obtained solution was subjected to HPLC (column: Shodex C18P-4E (produced by Showa Denko K. K.), column temperature: 40 °C, eluent: methanol / 10 mM potassium phosphate solution (pH 3) = 10/90, detection: UV 210 nm). It was confirmed that a peak originating from γ-butyrolactone disappeared completely and a single peak originating from sodium 4-hydroxybutanoate was obtained.

A supernatant of a culture solution was subjected to HPLC (column: Shodex SH-1011 (produced by Showa Denko K. K.), column temperature: 60 °C, eluent: 25 mM sulfuric acid aqueous solution, flow rate: 0.6 mL/min, detection: differential refraction detector). In the culture solution, 350 mg/L of 3-hydroxybutanoic acid was detected.

In the present embodiment, a compound that was added into a culture solution and had a 4-hydroxybutanoic acid backbone provided 4-hydroxybutyryl-CoA due to action of a gene that coded 4-hydroxybutyryl-CoA; CoA mutase indicated by sequence 3. Furthermore, conversion into 3-hydroxybutyryl-CoA was caused by action of a gene of sequence 1 and a gene of sequence 2. It was considered that converted 3-hydroxybutyryl-CoA was further discharged into a culture solution as 3-hydroxybutanoic acid due to action of thioesterase that was possessed by Escherichia coli that was a host.

### (Comparative Example 1)

An Escherichia coli Rosetta Blue (DE3) strain (produced by Novagen, Inc.) was transformed by using a plasmid pETSD3 obtained in Practical Example 1 and an ordinary method to obtain a transformed body pETSD3 / Rosetta Blue (DE3). An obtained transformed body was cultured by a method similar to Practical Example 1. A culture solution after culturing was subjected to HPLC similar to Practical Example 1 but 3-hydroxybotanic acid was not detected.

It was considered that genes of sequence 1 and sequence 2 were not possessed in a method in Comparative Example 1, and hence, there was no action of these genes so that 4-hydroxybutyryl-CoA was not converted into 3-hydroxybutyryl-CoA.

### (Comparative Example 2)

Inverse PCR of a plasmid pETSD123 obtained in Practical Example 1 was executed by using a primer for executing annealing and amplification outward in front and back of a code region of sequence 1 on pETSD123 to obtain a straight-chain blunt end fragment wherein sequence 1 was eliminated from pETSD123.

A blunt end fragment of an abfD gene sequence (that corresponded to 4-hydroxybutyryl-CoA dehydratase) derived from a Clostridium aminobutyricum wild strain indicated by sequence number 5 was totally synthesized by an ordinary method. An obtained blunt end fragment and a phosphorylation product of a straight-chain blunt end fragment described previously were subjected to ligation, transformation, PCR, and restriction enzyme treatment for mapping to obtain a plasmid pETSD523 with singly substituting and inserted sequence 5 in a direction identical to that of sequence 1.

An Escherichia coli Rosetta Blue (DE3) strain (produced by Novagen, Inc.) was transformed by using an obtained plasmid pETSD523 and an ordinary method to obtain a transferred body pETSD523/Rosetta Blue (DE3). An obtained transformed body pETSD523/Rosetta Blue (DE3) was cultured and evaluated in a method similar to Practical Example 1. In a culturing solution, 3-hydroxybutanoic acid was not detected.

It was considered that a gene of sequence 5 did not act in the present embodiment, and hence, conversion of 4-hydroxybutyryl-CoA into 3-hydroxybutyryl-CoA was not caused.

### (Practical Example 2)

A blunt end fragment of a base sequence indicated by sequence number 4 was totally synthesized by an ordinary method inserted into an SmaI site in mult-cloning sites of a cloning vector pUC18 to obtain a plasmid. Sequences that corresponded to 15 bp that included "CAT" at an upstream side and 15 bp that included "ATG" at a downstream side of an NdeI site CATATG in multi-cloning sites of pETSD132 obtained in Practical Example 1, in addition to respective 15 bp that corresponded to a 5'-end and 3'-end of sequence 4, were added to an outside of an obtained plasmid as a template, to prepare a primer with respective 30 bp at both ends thereof, and amplification reaction was caused to obtain a blunt end fragment that included sequence 4. An obtained fragment and a straight-chain fragment obtained by inverse PCR of pETSD123 at a center of a restriction enzyme site NdeI were subjected to ligation in In-Fusion Cloning to obtain a plasmid pETSD4123 that contained sequences 1 to 4.

An Escherichia coli Rosetta Blue (DE3) strain was transformed by using an obtained plasmid pETSD4123 and an ordinary method.

An obtained transformed body pETSD4123/Rosetta Blue (DE3) was aerobically cultured in 5 mL of an LB medium that contained 100 mg/L of ampicillin and 32 mg/L of chloramphenicol at 37 °C for 12 hours. 0.1 mL of a culture solution was implanted in 5 mL of the above-mentioned evaluation medium 1 that contained 100 mg/L of ampicillin, 32 mg/L of chloramphenicol, 0.2 mM of IPTG, and further 0.3% of sodium 4-hydroxybutanoate, and aerobically cultured at 30 °C for 48 hours. A supernatant of a culture solution was subjected to HPLC (column: Shodex SH-1011 (produced by Showa Denko K. K.), column temperature: 60 °C, eluent: 25 mM sulfuric acid aqueous solution, flow rate: 0.6 mL/min, detection: differential refraction detector). In the culture solution, 320 mg/L of 3-hydroxybutanoic acid, 240 mg/L of 1,3-butanediol, and 220 mg/L of 1,4-butanediol were detected.

In the present embodiment, a compound that was added into a culture solution and had a 4-hydroxybutanoic acid backbone provided 4-hydroxybutyryl-CoA due to action of a gene that coded 4-hydroxybutyryl-CoA; CoA mutase indicated by sequence 3. Furthermore, conversion into 3-hydroxybutyryl-CoA was caused by action of a gene of sequence 1 and a gene of sequence 2. It was considered that converted 3-hydroxybutyryl-CoA was such that 1,3-butanediol or 1,4-butanediol was produced from 3-hydroxybutyryl-CoA or 4-hydroxybutyryl-CoA, respectively, due to action of a gene that coded an aldehyde reductase of sequence 4, and further, 3-hydroxybutanoic acid was produced from 3-hydroxybutyryl-CoA and these were discharged into a culture solution, due to action of thioesterase that was possessed by Escherichia coli that was a host.

### (Comparative Example 3)

A plasmid pETSD4123 obtained in Practical Example 2 was treated with a restriction enzyme BamHI. Longer chain sides of two obtained fragments were excised and subjected to ligation to obtain a plasmid pETSD43 wherein gene sequences of sequences 1 and 2 were eliminated.

An Escherichia coli Rosetta Blue (DE3) strain was transformed by using an obtained plasmid pETSD43 and an ordinary method. An obtained transformed body was cultured by a method similar to Practical Example 2. In a culture solution after culturing, 290 mg/L of 1,4-butanediol was detected and 3-hydroxybutanoic acid or 1,3-butanediol was not detected.

In the present embodiment, a compound that was added into a culture solution and had a 4-hydroxybutanoic acid backbone provided 4-hydroxybutyryl-CoA due to action of a gene that coded 4-hydroxybutyryl-CoA; CoA mutase indicated by sequence 3. However, there was no action of a gene that had base sequences of sequences 1 and 2, and hence, conversion of 4-hydroxybutyryl-CoA into 3-hydroxybutyryl-CoA was not caused. It was considered that 3-hydroxybutanoic acid or 1,3-butanediol was not detected in a culture solution after culturing accordingly.

### (Practical Example 3)

According to a method similar to Practical Example 1 and Practical Example 2, T7 promoters were separately and upstream arranged in multi-cloning sites of a vector pET17b and sequence 4, sequence 1, and sequence 2 were inserted in this order to obtain a plasmid pETSD412.

Furthermore, a base sequence indicated by sequence number 6 and a base sequence indicated by sequence number 7 were similarly inserted into multi-cloning sites 1 and multi-cloning sites 2 of vector pCDFDuet (produced by Novagen, Inc.) to obtain a plasmid pCDSD6-7.

An Escherichia coli Rosetta Blue (DE3) strain was transformed by using obtained plasmids pETSD412 and pCDSD6-7 and an ordinary method.

An obtained transformed body pETSD412+pCDSD6-7/Rosetta Blue (DE3) was aerobically cultured in 5 mL of an LB medium that contained 100 mg/L of ampicillin, 50 mg/L of streptomycin, and 32 mg/L of chloramphenicol at 37 °C for 12 hours. 0.1 mL of a culture solution was implanted in 5 mL of the above-mentioned evaluation medium 1 that contained 100 mg/L of ampicillin, 50 mg/L of streptomycin, 32 mg/L of chloramphenicol, and 0.2 mM of IPTG, and aerobically cultured at 30 °C for 48 hours. A supernatant of a culture solution was subjected to HPLC (column: Shodex SH-1011 (produced by Showa Denko K. K.), column temperature: 60 °C, eluent: 25 mM sulfuric acid aqueous solution, flow rate: 0.6 mL/min, detection: differential refraction detector). In the culture solution, 410 mg/L of 3-hydroxybutanoic acid, 220 mg/L of 1,3-butanediol, and 40 mg/L of 1,4-butanediol were detected.

In the present embodiment, 3-hydroxybutyryl-CoA was produced due to action of a gene that had sequence 6 and sequence 7, while acetyl-CoA supplied from a glycolysis system was a substrate. Furthermore, a par of 3-hydroxybutyryl-CoA was converted into 4-hydroxybutyryl-CoA due to action of a gene of sequence 1 and a gene of sequence 2. It was considered that 1,3-butanediol or 1,4-butanediol was produced from 3-hydroxybutyryl-CoA or 4-hydroxybutyryl-CoA, respectively, due to a gene that coded an aldehyde reductase of sequence 4, and further, 3-hydroxybutanoic acid was produced from 3-hydroxybutyryl-CoA and these were discharged into a culture solution, due to action of thioesterase that was possessed by Escherichia coli that was a host.

### (Comparative Example 4)

According to a method similar to Practical Example 1 to Practical Example 3, T7 promoters were separately and upstream arranged in multi-cloning sites of a vector pET17b and sequence 4, sequence 5, and sequence 2 were inserted in this order to obtain a plasmid pETSD452.

Furthermore, a base sequence indicated by sequence number 6 and a base sequence indicated by sequence number 7 were similarly inserted into multi-cloning sites 1 and multi-cloning sites 2 of vector pCDFDuet (produced by Novagen, Inc.) to obtain a plasmid pCDSD6-7.

An Escherichia coli Rosetta Blue (DE3) strain was transformed by using obtained plasmids pETSD452 and pCDSD6-7 and an ordinary method.

A transformed body pETSD452+pCDSD6-7/Rosetta Blue (DE3) was aerobically cultured in 5 mL of an LB medium that contained 100 mg/L of ampicillin, 50 mg/L of streptomycin, and 32 mg/L of chloramphenicol at 37 °C for 12 hours. 0.1 mL of a culture solution was implanted in 5 mL of the above-mentioned evaluation medium 1 that contained 100 mg/L of ampicillin, 50 mg/L of streptomycin, 32 mg/L of chloramphenicol, and 0.2 mM of IPTG, and aerobically cultured at 30 °C for 48 hours. A supernatant of a culture solution was subjected to HPLC (column: Shodex SH-1011 (produced by Showa Denko K. K.), column temperature: 60 °C, eluent: 25 mM sulfuric acid aqueous solution, flow rate: 0.6 mL/min, detection: differential refraction detector). In the culture solution, 530 mg/L of 3-hydroxybutanoic acid and 270 mg/L of 1,3-butanediol were detected.

It was considered that a gene of sequence 5 did not act in the present embodiment, and hence, conversion of 4-hydroxybutyryl-CoA into 3-hydroxybutyryl-CoA was not caused, and as a result, 1,4-butanediol was not produced.

The present application claims priority based on Japanese Patent Application No. 2012-209982 filed on September 24, 2012 before the Japan Patent Office and the entire contents of Japanese Patent Application No. 2012-209982 are incorporated by reference in the present application.

## Claims

1. A gene described in any of undermentioned (a) - (c), wherein the gene in combination with a gene that codes enoyl-CoA hydratase is able to provide a microbe or a culture of the microbe with an ability to convert 3-hydroxybutyryl-CoA into 4-hydroxybutyryl-CoA or 4-hydroxybutyryl-CoA into 3-hydroxybutyryl-CoA.
(a) a gene that has a base sequence of sequence number 1
(b) a gene that has a base sequence such that one or more bases are deleted, substituted, or added in a base sequence of sequence number 1, wherein the gene has a base sequence with an identity greater than or equal to 90% with respect to the base sequence of sequence number 1
(c) a gene that hybridizes with a gene that has a base sequence complimentary with a gene that has a base sequence described in sequence number 1 on a stringent condition

2. The gene as claimed in claim 1, wherein the microbe is Escherichia coli, a yeast, a corynebacterium, or a clostridium.

3. A microbe that includes the gene as claimed in claim 1 and a gene that codes enoyl-CoA hydratase.

4. The microbe as claimed in claim 3, further including a gene that codes acyl-CoA reductase.

5. A conversion method, wherein the microbe as claimed in claim 3 or a culture of the microbe is used to convert 3-hydroxybutyryl-CoA into 4-hydroxybutyryl-CoA or 4-hydroxybutyryl-CoA into 3-hydroxybutyryl-CoA.

6. A manufacturing method, wherein the microbe as claimed in claim 4 or a culture of the microbe is used to manufacture 1,4-butanediol.

7. The manufacturing method as claimed in claim 6, wherein the microbe includes a gene that codes at least one of acetoacetyl-CoA synthase or acetoacetyl-CoA reductase.

8. The manufacturing method as claimed in claim 6, wherein 1,4-butanediol is manufactured from acetyl-CoA via 3-hydroxybutyryl-CoA.


SEQUENCE LISTING

<110>  Showa Denko K.K.
<120>  Gene, microorganism, conversion method and production method
<130>  P20120174 (SHD13008PCT)
<160>  7
<170>  PatentIn version 3.5

<210>  1
<211>  1473
<212>  DNA
<213>  Artificial Sequence
<220>
<223>  putative 4-hydroxybutyryl-CoA dehydratase gene
<220>
<223>  Inventor: Aoki, Hirobumi\201G Kokido, Yuzuru; Yoneda, Tadashi
<400>  1
atgctgatga ccgccgaaca gtacatcgaa agcctgcgca agctgaacac gcgtgtctac
60

atgtttggtg aaaagatcga gaactgggta gaccatccca tgattcgccc ttcgatcaat
120

tgtgtggcaa tgacctatga actggcgcaa gatccccagt atgccgattt gatgaccacg
180

aaatcgaatc tgattggcaa gacgattaat cgcttcgcga acttacacca aagcaccgat
240

gatctgcgca agaaagtcaa aatgcaacgt ctgctgggcc agaaaaccgc gagttgcttt
300

cagcggtgtg ttgggatgga tgcgttcaat gccgtgtttt ccaccactta tgagattgat
360

cagaaatacg ggactaacta ccacaagaac ttcacggagt atctgaaata tattcaagaa
420

aatgacttga tcgtggatgg tgctatgacc gacccgaaag gcgaccgtgg gctggcaccg
480

agtgcccaga aagacccaga tctgtttctg cgcatcgtgg agaaacgcga ggatggtatc
540

gtagtccgtg gtgcgaaagc acatcaaacg ggctcaatta atagccatga gcacatcatt
600

atgccgacca ttgctatgac agaagcggat aaagactatg cggttagttt tgcttgtcca
660

tccgatgcgg atggtttgtt tatgatctat ggtcgccagt cttgcgacac ccgcaaaatg
720

gaggaaggcg cagatattga cttaggcaac aaacagtttg gcggccaaga agcccttgtc
780

gtgtttgaca acgtattcat cccgaatgat cgcattttct tatgccagga atacgatttt
840

gcgggaatga tggtcgaacg cttcgctggt tatcatcgcc agtcttacgg tggctgcaaa
900

gtgggtgtgg gtgacgtcgt tattggagcc gcagcccttg cagcggatta caacggtgcc

960

cagaaggcca gccatgtgaa agacaaactg atcgaaatga cacacttgaa cgaaaccctg
1020

tattgttgtg ggattgcgtg ctcagccgaa ggttatccga ctgcagccgg caattaccag
1080

atcgatctcc tccttgcgaa tgtgtgcaaa cagaacatta cgcgttttcc gtacgagatt
1140

gtacgtctgg cggaagatat tgccggagga ctgatggtta ctatgcctag cgaagcggac
1200

ttcaaaagcg aaacggttgt tgggcgtgat ggcgaaacca ttggcgattt ctgcaacaaa
1260

ttctttgcgg cagcaccgac ttgcaccaca gaagaacgga tgcgtgtgct ccgctttctg
1320

gagaacattt gtttaggcgc atccgctgtt gggtatcgta cagaatcgat gcatggtgcg
1380

ggctctccac aggctcaacg catcatgatt gcccgtcaag gcaatattaa cgcgaagaaa
1440

gaactcgcta aagcgatcgc tggcatcaaa taa                              1473


<210>  2
<211>  405
<212>  DNA
<213>  Artificial Sequence
<220>
<223>  Aeromonas caviae phaJ gene
<400>  2
atgagcgcac aatccctgga agtaggccag aaggcccgtc tcagcaagcg gttcggggcg
60

gcggaggtag ccgccttcgc cgcgctctcg gaggacttca accccctgca cctggacccg
120

gccttcgccg ccaccacggc gttcgagcgg cccatagtcc acggcatgct gctcgccagc
180

ctcttctccg ggctgctggg ccagcagttg ccgggcaagg ggagcatcta tctgggtcaa
240

agcctcagct tcaagctgcc ggtctttgtc ggggacgagg tgacggccga ggtggaggtg
300

accgcccttc gcgaggacaa gcccatcgcc accctgacca cccgcatctt cacccaaggc
360

ggcgccctcg ccgtgacggg ggaagccgtg gtcaagctgc cttaa                 405


<210>  3
<211>  1290
<212>  DNA
<213>  Artificial Sequence
<220>
<223>  Clostridium kluyveri CAT2 gene

<400> 3
atggagtggg aagagatata taaagagaaa ctggtaactg cagaaaaagc tgtttcaaaa
60

atagaaaacc atagcagggt agtttttgca catgcagtag gagaacccgt agatttagta
120

aatgcactag ttaaaaataa ggataattat ataggactag aaatagttca catggtagct
180

atgggcaaag gtgtatatac aaaagagggt atgcaaagac attttagaca taatgctttg
240

tttgtaggcg gatctactag agatgcagta aattcaggaa gagcagttta tacaccttgt
300

tttttctatg aagtgccaag tttgtttaaa gaaaaacgtt tgcctgtaga tgtagcactt
360

attcaggtaa gtgagccaga taaatatggc tactgcagtt ttggagtttc caatgactat
420

accaagccag cagcagaaag tgctaagctt gtaattgcag aagtgaataa aaacatgcca
480

agaactcttg gagattcttt tatacatgta tcagatattg attatatagt ggaagcttca
540

cacccattgt tagaattgca gcctcctaaa ttgggagatg tagaaaaagc cataggagaa
600

aactgtgcat ctttaattga agatggagct actcttcagc ttggaatagg tgctatacca
660

gatgcggtac ttttattctt aaagaacaaa aagaatttag gaatacattc tgagatgata
720

tcagatggtg tgatggaact ggtgaaggca ggggttatca ataacaagaa aaagaccctc
780

catccaggca aaatagttgt aacattttta atgggaacaa aaaaattata tgattttgta
840

aacaataatc caatggtaga aacttattct gtagattatg taaataatcc actggtaatt
900

atgaaaaatg acaatatggt ttcaataaat tcttgtgttc aagtagactt aatgggacaa
960

gtatgttctg aaagtatagg attgaaacag ataagtggag tgggaggcca ggtagatttt
1020

attagaggag ctaatctatc aaagggtgga aaggctatta tagctatacc ttccacagct
1080

ggaaaaggaa aagtttcaag aataactcca cttctagata ctggtgctgc agttacaact
1140

tctagaaatg aagtagatta tgtagttact gaatatggtg ttgctcatct taagggcaaa
1200

actttaagaa atagggcaag agctctaata aatatcgctc atccaaaatt cagagaatca
1260

ttaatgaatg aatttaaaaa gagattttag 1290

<210> 4
<211> 1407
<212> DNA
<213> Artificial Sequence
<220>
<223> Clostridium beijerinckii ald gene
<400> 4
atgaataaag acacactaat acctacaact aaagatttaa aagtaaaaac aaatggtgaa
60

aacattaatt taaagaacta caaggataat tcttcatgtt tcggagtatt cgaaaatgtt
120

gaaaatgcta taagcagcgc tgtacacgca caaaagatat tatcccttca ttatacaaaa
180

gagcaaagag aaaaaatcat aactgagata agaaaggccg cattacaaaa taaagaggtc
240

ttggctacaa tgattctaga agaaacacat atgggaagat atgaggataa aatattaaaa
300

catgaattgg tagctaaata tactcctggt acagaagatt taactactac tgcttggtca
360

ggtgataatg gtcttacagt tgtagaaatg tctccatatg gtgttatagg tgcaataact
420

ccttctacga atccaactga aactgtaata tgtaatagca taggcatgat agctgctgga
480

aatgctgtag tatttaacgg acacccatgc gctaaaaaat gtgttgcctt tgctgttgaa
540

atgataaata aggcaattat ttcatgtggc ggtcctgaaa atctagtaac aactataaaa
600

aatccaacta tggagtctct agatgcaatt attaagcatc cttcaataaa acttctttgc
660

ggaactgggg gtccaggaat ggtaaaaacc ctcttaaatt ctggtaagaa agctataggt
720

gctggtgctg gaaatccacc agttattgta gatgatactg ctgatataga aaaggctggt
780

aggagcatca ttgaaggctg ttcttttgat aataatttac cttgtattgc agaaaaagaa
840

gtatttgttt ttgagaatgt tgcagatgat ttaatatcta acatgctaaa aaataatgct
900

gtaattataa atgaagatca agtatcaaaa ttaatagatt tagtattaca aaaaaataat
960

gaaactcaag aatactttat aaacaaaaaa tgggtaggaa aagatgcaaa attattctta
1020

gatgaaatag atgttgagtc tccttcaaat gttaaatgca taatctgcga agtaaatgca

1080

aatcatccat ttgttatgac agaactcatg atgccaatat tgccaattgt aagagttaaa
1140

gatatagatg aagctattaa atatgcaaag atagcagaac aaaatagaaa acatagtgcc
1200

tatatttatt ctaaaaatat agacaaccta aatagatttg aaagagaaat agatactact
1260

atttttgtaa agaatgctaa atcttttgct ggtgttggtt atgaagcaga aggatttaca
1320

actttcacta ttgctggatc tactggtgag ggaataacct ctgcaaggaa ttttacaaga
1380

caaagaagat gtgtacttgc cggctaa                                    1407

<210>  5
<211>  1473
<212>  DNA
<213>  Artificial Sequence
<220>
<223>  Clostridium aminobutylicum abfD gene
<400>  5
atgttaatga cagcagaaca gtacattgag agtctaagaa agctaaacac aagagtttat
60

atgtttggtg aaaaaatcga gaattgggtg gatcatccaa tgatcagacc ttccatcaac
120

tgcgtagcaa tgacttatga attagctcag gatcctcagt acgctgactt aatgactaca
180

aagtcaaact taataggtaa aactatcaac agatttgcaa atctacacca gagcacagat
240

gaccttagaa aaaaggttaa gatgcagaga cttcttggac agaagaccgc atcatgcttc
300

cagagatgtg taggtatgga cgctttcaat gcagttttct caactacata tgaaatcgac
360

cagaaatatg gaacaaacta tcacaagaac tttactgaat acttaaagta tatacaggaa
420

aatgacctta ttgttgacgg tgcaatgact gaccctaagg gtgacagagg acttgctcca
480

tccgcacaga aggatccaga tcttttcttg agaatcgttg aaaaaagaga agatggtatc
540

gttgtaagag gagctaaggc tcaccagact ggttccatca actcccacga acacatcatc
600

atgcctacaa tcgctatgac agaagctgat aaggattatg cagtatcatt tgcttgtcct
660

tccgatgctg atggtctatt catgatctac ggcagacagt catgtgacac aagaaagatg
720

gaagaaggcg ctgacattga ccttggtaac aagcagttcg gcggacagga agctttagtc
780

gtattcgata acgtatttat tccaaatgac agaatcttcc tttgccaaga atatgatttc
840

gctggcatga tggtagaaag atttgctgga taccacagac agtcatacgg cggatgtaag
900

gttggagtag gcgacgttgt aatcggtgct gctgctttag ctgctgacta caatggagct
960

cagaaggctt ctcacgttaa agataagctt atcgaaatga ctcacttaaa tgaaacttta
1020

tattgctgcg gtattgcttg ttcagcagaa ggttatccaa ctgctgctgg taactatcag
1080

attgaccttc ttcttgcaaa tgtatgtaag cagaacatca ctagattccc ttacgaaatc
1140

gtaagactag ctgaagatat cgctggtgga ttaatggtta ctatgccttc agaagctgac
1200

tttaagtcag aaacagttgt tggtagagat ggcgaaacta ttggagattt ctgcaataag
1260

ttcttcgctg ctgctcctac ttgcacaaca gaagaaagaa tgagagttct tagattctta
1320

gaaaacatct gcttaggtgc atccgctgta ggttacagaa ctgaatccat gcatggtgca
1380

ggttcccctc aggctcagag aatcatgatc gctcgtcagg gcaacatcaa cgctaagaaa
1440

gaattagcta aggcaatcgc tggaattaaa taa                              1473


<210>  6
<211>  1182
<212>  DNA
<213>  Artificial Sequence
<220>
<223>  Ralstonia eutropha phaA gene
<400>  6
atgactgacg ttgtcatcgt atccgccgcc cgcaccgcgg tcggcaagtt tggcggctcg
60

ctggccaaga tcccggcacc ggaactgggt gccgtggtca tcaaggccgc gctggagcgc
120

gccggcgtca agccggagca ggtgagcgaa gtcatcatgg gccaggtgct gaccgccggt
180

tcgggccaga accccgcacg ccaggccgcg atcaaggccg gcctgccggc gatggtgccg
240

gccatgacca tcaacaaggt gtgcggctcg ggcctgaagg ccgtgatgct ggccgccaac
300

gcgatcatgg cgggcgacgc cgagatcgtg gtggccggcg gccaggaaaa catgagcgcc
360

gccccgcacg tgctgccggg ctcgcgcgat ggtttccgca tgggcgatgc caagctggtc
420

gacaccatga tcgtcgacgg cctgtgggac gtgtacaacc agtaccacat gggcatcacc
480

gccgagaacg tggccaagga atacggcatc acacgcgagg cgcaggatga gttcgccgtc
540

ggctcgcaga acaaggccga agccgcgcag aaggccggca agtttgacga agagatcgtc
600

ccggtgctga tcccgcagcg caagggcgac ccggtggcct tcaagaccga cgagttcgtg
660

cgccagggcg ccacgctgga cagcatgtcc ggcctcaagc ccgccttcga caaggccggc
720

acggtgaccg cggccaacgc ctcgggcctg aacgacggcg ccgccgcggt ggtggtgatg
780

tcggcggcca aggccaagga actgggcctg accccgctgg ccacgatcaa gagctatgcc
840

aacgccggtg tcgatcccaa ggtgatgggc atgggcccgg tgccggcctc caagcgcgcc
900

ctgtcgcgcg ccgagtggac cccgcaagac ctggacctga tggagatcaa cgaggccttt
960

gccgcgcagg cgctggcggt gcaccagcag atgggctggg acacctccaa ggtcaatgtg
1020

aacggcggcg ccatcgccat cggccacccg atcggcgcgt cgggctgccg tatcctggtg
1080

acgctgctgc acgagatgaa gcgccgtgac gcgaagaagg gcctggcctc gctgtgcatc
1140

ggcggcggca tgggcgtggc gctggcagtc gagcgcaaat aa                    1182


<210>  7
<211>  741
<212>  DNA
<213>  Artificial Sequence
<220>
<223>  Ralstonia eutropha phaB gene
<400>  7
atgactcagc gcattgcgta tgtgaccggc ggcatgggtg gtatcggaac cgccatttgc
60

cagcggctgg ccaaggatgg ctttcgtgtg gtggccggtt gcggccccaa ctcgccgcgc
120

cgcgaaaagt ggctggagca gcagaaggcc ctgggcttcg atttcattgc ctcggaaggc
180

aatgtggctg actgggactc gaccaagacc gcattcgaca aggtcaagtc cgaggtcggc
240

gaggttgatg tgctgatcaa caacgccggt atcacccgcg acgtggtgtt ccgcaagatg

300

acccgcgccg actgggatgc ggtgatcgac accaacctga cctcgctgtt caacgtcacc
360

aagcaggtga tcgacggcat ggccgaccgt ggctggggcc gcatcgtcaa catctcgtcg
420

gtgaacgggc agaagggcca gttcggccag accaactact ccaccgccaa ggccggcctg
480

catggcttca ccatggcact ggcgcaggaa gtggcgacca agggcgtgac cgtcaacacg
540

gtctctccgg gctatatcgc caccgacatg gtcaaggcga tccgccagga cgtgctcgac
600

aagatcgtcg cgacgatccc ggtcaagcgc ctgggcctgc cggaagagat cgcctcgatc
660

tgcgcctggt tgtcgtcgga ggagtccggt ttctcgaccg gcgccgactt ctcgctcaac
720

ggcggcctgc atatgggctg a                                           741